# FASCICULE DE BREVET EUROPEEN

(11) **EP 1 417 184 B1**
(45) Date de publication et mention de la délivrance du brevet: **29.08.2007**
(21) Numéro de dépôt: 02772490.5
(22) Date de dépôt: 07.08.2002
(51) Int. Cl.: C07D 243/08, A61Q 5/10, A61K 8/49

(54) **COMPOSITION POUR LA TEINTURE DE FIBRE KERATINIQUES CONTENANT UNE PARAPHENYLENEDIAMINE SUBSTITUEE PAR UN RADICAL DIAZACYCLOHEPTANE**
ZUSAMMENSETZUNG ZUM FÄRBEN VON KERATINÖSEN FASERN, ENTHALTEND EIN MIT EINEM DIAZACYCLOHEPTANREST SUBSTITUIERTES PARAPHENYLENDIAMIN
COMPOSITION FOR DYEING KERATINOUS FIBRES CONTAINING A PARAPHENYLENEDIAMINE SUBSTITUTED BY A DIAZACYCLOHEPTANE RADICAL

(30) Priorité: 08.08.2001 FR 0110597
(43) Date de publication de la demande: 12.05.2004
(73) Titulaire: L'ORÉAL, 75008 Paris (FR)
(72) Inventeur: SABELLE, Stéphane, F-75005 Paris (FR); TERRANOVA, Eric, F-06520 Magagnosc (FR)
(74) Mandataire: Wattremez, Catherine
(86) Numéro de dépôt international: PCT/FR2002/002822
(87) Numéro de publication internationale: WO 2003/014093

(56) Documents cités:
- EP-A- 0 962 452
- WO-A-02/06278
- WO-A-98/38175
- WO-A-98/58926
- JP-A- 11 139 969

## Description

L'invention a pour objet une composition pour la teinture par oxydation des fibres kératiniques, en particulier des cheveux humains, comprenant une base d'oxydation du type paraphénylènediamine substituée par un radical diazacycloheptane. L'invention concerne aussi le procédé de teinture des fibres mettant en oeuvre cette composition, ainsi que les composés nouveaux du type précédemment cité.

Il est connu de teindre les fibres kératiniques et en particulier les cheveux humains avec des compositions tinctoriales contenant des précurseurs de colorant d'oxydation, appelés généralement bases d'oxydation, tels que des ortho ou paraphénylènediamines, des ortho ou paraaminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des modificateurs de coloration appelés généralement coupleur, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est déjà connu d'utiliser des dérivés 1,4-diazacycloheptane pour la coloration par oxydation de fibres kératiniques. Par exemple, le brevet US 6 165 230 décrit une composition tinctoriale contenant des dérivés 1,4-diazacycloheptane substitués sur les deux atomes d'azote du cycle par un groupement aminobenzène qui permet d'obtenir des colorations intenses.

Le but de la présente invention est de fournir de nouvelles compositions pour la teinture de fibres kératiniques ne présentant pas les inconvénients de celles de la technique antérieure. En particulier, le but de la présente invention est de fournir des compositions qui présentent des teintures puissantes, peu sélectives et particulièrement résistantes, tout en étant capables d'engendrer des colorations intenses dans des nuances variées, en particulier dans les nuances fondamentales.

Ce but est atteint avec la présente invention qui a pour objet une composition pour la teinture par oxydation des fibres kératiniques comprenant une base d'oxydation de formule (I) suivante et/ou les sels d'addition correspondants : 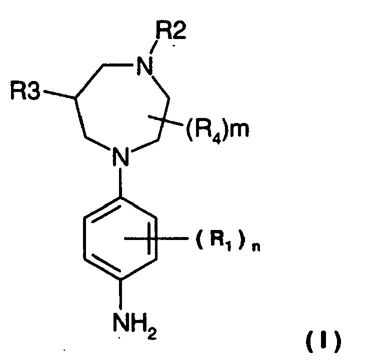 dans laquelle :
- **R₁** représente
   - un atome d'halogène, de préférence chlore ou brome ;
   - une chaîne hydrocarbonée en C₁-C₆, saturée ou pouvant contenir une ou plusieurs liaisons doubles et/ou une ou plusieurs liaisons triples, linéaire ou ramifiée pouvant former un cycle ayant de 3 à 6 chaînons, un ou plusieurs atomes de carbone pouvant être remplacés par un atome d'oxygène, d'azote ou de soufre, par un groupement SO₂ ou lorsque le carbone est terminal par un atome d'halogène, de préférence un atome de chlore ou de brome ; ledit radical R₁ ne comportant pas de liaison peroxyde, ni de radicaux diazo, nitro ou nitroso ;
- **n** est compris entre 0 et 4 inclus, étant entendu que lorsque n est supérieur ou égal à 2 alors les radicaux R₁ peuvent être identiques ou différents,
- **R₂** représente
   - un atome d'hydrogène ;
   - un radical alkyle pouvant être insaturé, non substitué ou substitué par un ou plusieurs radicaux carboxylique, alkylcarbonyle, alcoxycarbonyle, carbamoyle, mono- ou di-alkylcarbamoyle, hétérocyclique azoté, oxygéné et/ou soufré, saturé et/ou insaturé à 4, 5, 6 ou 7 atomes ;
   - un radical alkyle pouvant être insaturé, substitué en position 2 ou plus par un ou plusieurs radicaux hydroxy, alcoxy, amino, mono- ou di-alkylamino, thiol ou halogène ;
   - un radical alkylcarbonyle ;
   - un radical alkoxycarbonyle ;
   - un radical mono- ou di-alkylcarbamoyle ;
   - un radical carbamoyle ;
   - un radical R₆R₇N-C=NR₅- où R₅, R₆ et R₇ représentent un hydrogène, un radical alkyle en C₁-C₄ ou un radical hydroxyalkyle, de préférence R₅ est l'hydrogène et R₆ et R₇ sont choisis parmi l'hydrogène ou un méthyle,
- **R₃** représente
   - un atome d'hydrogène,
   - un radical alkyle pouvant être insaturé ;
   - un radical hydroxy,
   - un radical hydroxyalkyle ;
   - un radical alcoxy ;
   - un radical alcoxyalkyle ;
   - un radical alkylcarbonyle ;
   - un radical hydroxyalcoxyalkyle ;
   - un radical amino,
   - un radical moalkylamino ou dialkylamino ;
   - un radical aminoalkyle, l'amine pouvant être mono ou disubstitué par un radical alkyle, acétyle ou hydroxyalkyle ;
   - un radical hydroxy- et amino- alkyle ;
   - un radical carboxyle ;
   - un radical carboxyalkyle ;
   - un radical carbamoyle ;
   - un radical carbamoylalkyle ;
   - un radical alkoxycarbonyle ;
   - un radical mono- ou dialkyl- aminocarbonyle ;
- **R₄** représente
   - un radical alkyle pouvant être insaturé ;
   - un radical hydroxyalkyle ;
   - un radical alcoxyalkyle ;
   - un radical alkylcarbonyle ;
   - un radical hydroxyalcoxyalkyle ;
   - un radical aminoalkyle, l'amine pouvant être mono ou disubstitué par un radical alkyle, acétyle, hydroxyalkyle ;
   - un radical hydroxy- et amino- alkyle ;
   - un radical carboxyle ;
   - un radical carboxyalkyle ;
   - un radical carbamoyle ;
   - un radical carbamoylalkyle ;
   - un radical alkoxycarbonyle ;
   - un radical mono- ou dialkyl- aminocarbonyle ;
- **m** est compris entre 0 et 4 inclus, étant entendu que lorsque m est supérieur ou égal à 2 alors les radicaux R₄ peuvent être identiques ou différents.

Les composés de formule (I) sont des paraphénylène diamines substitutées par un substituant du type 1,4-diazacycloheptane, substituant aussi appelé dans la littérature 1,4-diazépane.

Dans les définitions ci dessus, les radicaux ou groupes alkyle sont linéaires ou ramifiés et comprennent, sauf autre indication, de 1 à 10 atomes de carbone, de préférence 1 à 6 atomes de carbone. Un radical alcoxy est un radical alkyl-O, le groupe alkyle étant tel que défini précédemment.

On entend par un radical alkyle insaturé, un alkyle de 2 à 10 atomes de carbone et comprenant une ou plusieurs doubles et/ou triples liaisons.

Un radical alkyle substitué est un alkyle mono ou polysubstitué. Par exemple, un hydroxyalkyle ou un aminoalkyle est un alkyle qui peut être substitué par un ou plusieurs groupe hydroxy ou amino. Un radical alkyle substitué en position 2 ou plus est un alkyle de formule -CH₂-R, R étant un alkyle substitué.

Selon l'invention, lorsque qu'il est indiqué qu'un ou plusieurs des atomes de carbone du radical R₁ peuvent être remplacés par un atome d'oxygène, d'azote, ou de soufre ou par un groupement SO₂, et/ou que ledit radical **R₁** peut contenir une ou plusieurs liaisons doubles et/ou une ou plusieurs liaisons triples, cela signifie que l'on peut, à titre d'exemple, faire les transformations suivantes :

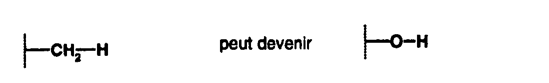

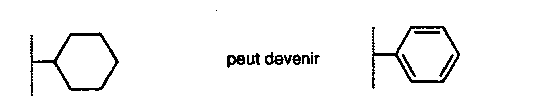

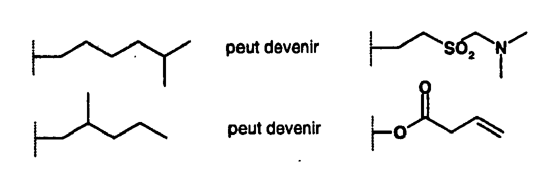

Lorsque n est égal à 0, le cycle benzénique est NH₂-C₆H₄-N-.

Dans la formule (I), lorsque n est différent de zéro, le radical R₁ est par exemple choisi parmi un atome de chlore, un radical méthyle, éthyle, isopropyle, vinyle, allyle, méthoxyméthyle, hydroxyméthyle, 1-carboxyméthyle, 1-aminométhyle, 2-carboxyéthyle, 2-hydroxyéthyle, 3-hydroxypropyle, 1,2-dihydroxyéthyle, 1-hydroxy-2-aminoéthyle, 1-amino-2-hydroxy éthyle, 1,2-diaminoéthyle, méthoxy, éthoxy, allyloxy, 2-hydroxyéthyloxy.

Lorsque n est différent de zéro, R₁ est de préférence un radical alkyle, hydroxyalkyle ou aminoalkyle, alcoxy, hydroxyalcoxy. Dans ce cas, R₁ peut être choisi parmi un radical méthyle, hydroxyméthyle, 2-hydroxyéthyle, 1,2-dihydroxyéthyle, méthoxy, 2-hydroxyéthoxy et plus préférentiellement, un radical méthyle, un radical hydroxyméthyle ou un radical 1,2-dihydroxyéthyle.

Selon un mode de réalisation préféré, les bases d'oxydations de formule (I) sont telles que n est égal à 0 ou 1.

Selon un mode de réalisation particulier de l'invention, le radical R₂ est choisi parmi l'hydrogène, un radical alkyle, un radical alkyle substitué par un hétérocyclique azoté, oxygéné et/ou soufré, saturé ou insaturé, à 4, 5 ,6 ou 7 atomes, un radical alcoxycarbonyle, un radical alkyle substitué en position 2 ou plus par un ou plusieurs radicaux hydroxy. Dans ce cas, R₂ peut être choisi parmi le radical 2-hydroxyéthyle, le radical 3 (1-pyrrolidinyl)propyle, le radical méthyle, le radical acétyle, l'hydrogène, et de façon plus préférentielle le radical 2-hydroxyéthyle, le radical méthyle ou l'hydrogène.

Selon un mode de réalisation particulier de l'invention, R₃ est choisi parmi l'hydrogène, un radical alkyle, un radical alkyle substitué par un ou plusieurs hydroxy, un radical alkyle substitué par un ou plusieurs amino, un radical carboxyle. Dans ce cas, R₃ peut être choisi parmi l'hydrogène, le radical hydroxyle, le radical carboxyle, le radical amino, le radical hydroxyméthyle, le radical aminométhyle. Parmi ces substituants, R₃ représente plus préférentiellement un radical hydrogène.

Selon un mode de réalisation particulier, R₄ est choisi parmi l'hydrogène, un radical alkyle, un radical alkyle substitué par un ou plusieurs hydroxy, un radical alkyle substitué par un ou plusieurs amino, un radical carboxyle. R₄ représente de façon préférentielle l'hydrogène.

Le carbone substitué par R₃ ou par R₄, peut être de configuration (R) et/ou (S)

Les sels d'addition correspondant à la formule (I) peuvent être des sels d'addition avec un acide ou des sels d'addition avec une base.

Parmi les bases d'oxydation de formule (I), on peut citer les composés suivants ou leurs sels d'addition avec un acide ou une base:

| formule | Nomenclature | formule | nomenclature |
|---|---|---|---|
| 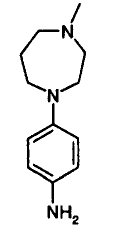 | 4-(4-Méthyl-[1,4]diazepan-1-yl)-phénylamine | 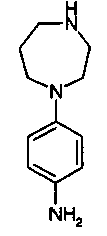 | 4-[1,4]Diazepan-1-yl-phénylamine |
| 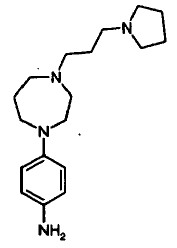 | 4-[4-(3-Pyrrolidin-1-yl-propyl)-[1,4]diazepan-1-yl]-phénylamine | 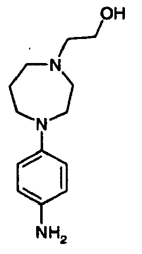 | 2-[4-(4-Amino-phényl)-[1,4]diazepan-1-yl]-ethanol |
| 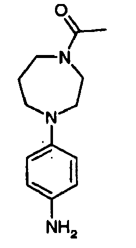 | 1-[4-(4-Amino-phényl)-[1,4]diazepan-1-yl]-ethanone | 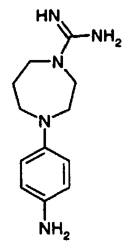 | 4-(4-Amino-phényl)-[1,4]diazepane-1-carboxamidine |
| 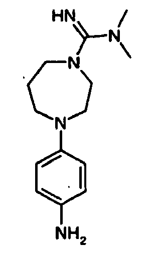 | 4-(4-Amino-phényl)-N,N-diméthyl-[1,4]diazepane-1-carboxamidine | 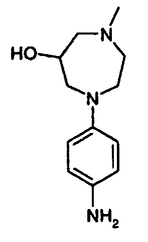 | 1-(4-Amino-phényl)-4-méthyl-[1,4]diazepan-6-ol |
| 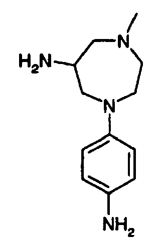 | 1-(4-Amino-phényl)-4-méthyl-[1,4]diazepan-6-ylamine | 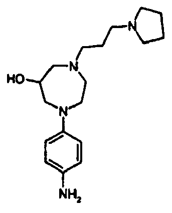 | 1-(4-Amino-phényl)-4-(3-pyrrolidin-1-yl-propyl)-[1,4]diazepan-6-ol |
| 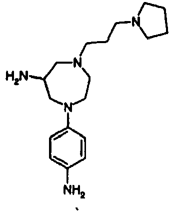 | 1-(4-Amino-phényl)-4-(3-pyrrolidin-1-yl-propyl)-[1,4]diazepan-6-ylamine | 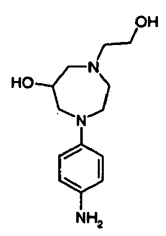 | 1-(4-Amino-phényl)-4-(2-hydroxy-ethyl)-[1,4]diazepan-6-ol |
| 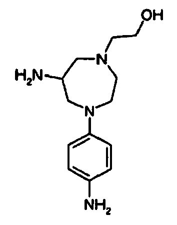 | 2-[6-Amino-4-(4-amino-phényl)-[1,4]diazepan-1-yl]-ethanol | 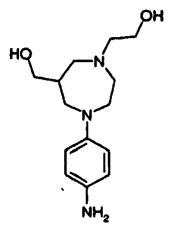 | 2-[4-(4-Amino-phényl)-6-hydroxyméthyl-[1,4]diazepan-1-yl]-ethanol |
| 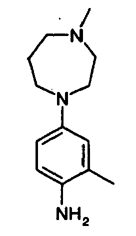 | 2-Méthyl-4-(4-méthyl-[1,4]diazepan-1-yl)-phénylamine | 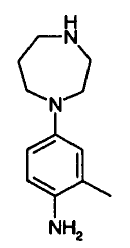 | 4-[1,4]Diazepan-1-yl-2-méthyl-phénylamine |
| 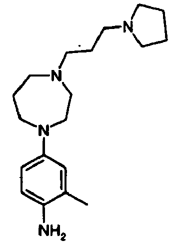 | 2-Méthyl-4-[4-(3-pyrrolidin-1-yl-propyl)-[1,4]diazepan-1-yl]-phénylamine | 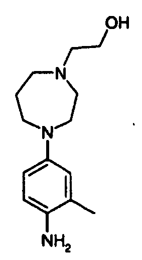 | 2-[4-(4-Amino-3-méthyl-phényl)-[1,4]diazepan-1-yl]-ethanol |
| 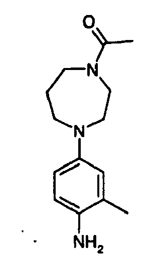 | 1-[4-(4-Amino-3-méthyl- phényl)-[1,4]diazepan-1-yl]-ethanone | 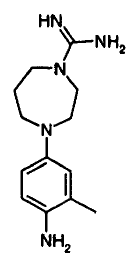 | 4-(4-Amino-3-méthyl-phényl)-[1,4]diazepane-1-carboxamidine |
| 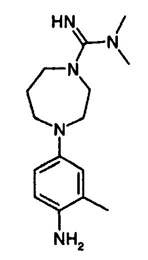 | 4-(4-Amino-3-méthyl-phényl)-N,N-diméthyl-[1,4]diazepane-1-carboxamidine | 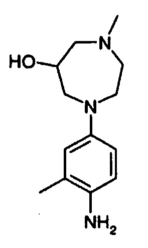 | 1-(4-Amino-3-méthyl-phényl)-4-méthyl-[1,4]diazepan-6-ol |
| 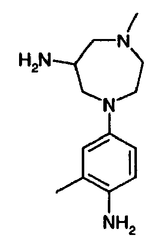 | 1-(4-Amino-3-méthyl-phényl)-4-méthyl-[1,4]diazepan-6-ylamine | 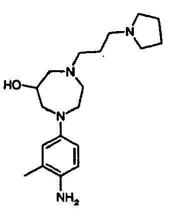 | 1-(4-Amino-3-méthyl-phényl)-4-(3-pyrrolidin-1-yl-propyl)-[1,4]diazepan-6-ol |
| 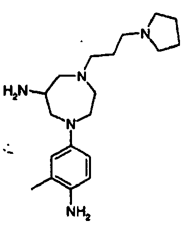 | 1-(4-Amino-3-méthyl-phényl)-4-(3-pyrrolidin-1-yl-propyl)-[1,4]diazepan-6-ylamine | 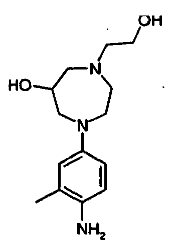 | 1-(4-Amino-3-méthyl-phényl)-4-(2-hydroxy-ethyl)-[1,4]diazepan-6-ol |
| 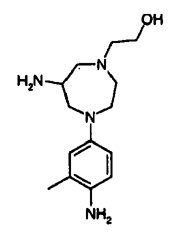 | 2-[6-Amino-4-(4-amino-3-méthyl-phényl)-[1,4]diazepan-1 -yl]-ethanol | 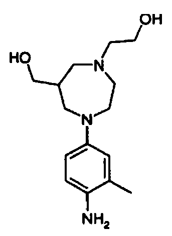 | 2-[4-(4-Amino-3-méthyl-phényl)-6-hydroxyméthyl-[1,4]diazepan-1-yl]-ethanol |

De préférence, les bases d'oxydation de formule (I) sont choisies parmi les composés suivants ou leurs sels d'addition avec un acide ou une base
- la 4-(4-Méthyl-[1,4]diazepan-1-yl)-phénylamine
- la 4-[1,4]Diazepan-1-yl-phénylamine
- la 4-[4-(3-Pyrrolidin-1-yl-propyl)-[1,4]diazepan-1-yl]-phénylamine
- la 2-[4-(4-Amino-phényl)-[1,4]diazepan-1-yl]-ethanol
- la 4-[1,4]Diazepan-1-yl-2-méthyl-phénylamine
- 1-[4-(4-Amino-phényl)-[1,4]diazepan-1-yl]-ethanone
- 4-(4-Amino-phényl)-[1,4]diazepane-1-carboxamidine
- 4-(4-Amino-phényl)-N,N-diméthyl-[1,4]diazepane-1-carboxamidine
- 1-(4-Amino-phényl)-4-méthyl-[1,4]diazepan-6-ol
- 2-Méthyl-4-(4-méthyl-[1,4]diazepan-1-yl)-phénylamine
- 2-Méthyl-4-[4-(3-pyrrolidin-1-yl-propyl)-[1,4]diazepan-1-yl]-phénylamine
- 2-[4-(4-Amino-3-méthyl-phényl)-[1,4]diazepan-1-yl]-ethanol
- 1-[4-(4-Amino-3-méthyl-phényl)-[1,4]diazepan-1-yl]-ethanone
- 4-(4-Amino-3-méthyl-phényl)-[1,4]diazepane-1-carboxamidin
- 4-(4-Amino-3-méthyl-phényl)-N,N-diméthyl-[1,4]diazepane-1-carboxamidine
- 1-(4-Amino-3-méthyl-phényl)-4-méthyl-[1,4]diazepan-6-ol.

Dans la composition de la présente invention, la ou les bases d'oxydation de formule (I) sont en général présente en quantité comprise entre 0,001 et 10 % en poids environ du poids total de la composition tinctoriale et préférentiellement entre 0,005 et 6 %.

Les composés de la présente invention peuvent être obtenus par analogie avec des procédés de préparation décrits dans la littérature, voir notamment la demande de brevet DE 4 241 532 (AGFA).

La composition de la présente invention peut en outre comprendre une ou plusieurs bases d'oxydation additionnelles. Ces bases d'oxydation additionnelles sont choisies parmi les bases d'oxydation classiquement utilisées en teinture d'oxydation, par exemple les paraphénylènediamines, les bis-phénylalkylènediamines, les paraaminophénols, les ortho-aminophénols et les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4-aminophénylpyrrolidine, le 2-thiényl paraphénylènediamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthytoxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le paraaminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre, d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Les bases d'oxydation additionnelles sont en général présentes en quantité comprise entre 0,001 à 10 % en poids environ du poids total de la composition tinctoriale, et préférentiellement de 0,005 à 6 %.

La composition selon l'invention peut contenir un ou plusieurs coupleurs conventionnellement utilisés pour la teinture de fibres kératiniques. Parmi ces coupleurs, on peut notamment citer les métaphénylènediamines, les métaaminophénols, les métadiphénols, les coupleurs naphtaléniques et les coupleurs hétérocycliques et leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans la composition de la présente invention, le ou les coupleurs sont généralement présents en quantité comprise entre 0,001 et 10 % en poids environ du poids total de la composition tinctoriale et préférentiellement de 0,005 à 6 %.

D'une manière générale, les sels d'addition avec un acide utilisables dans le cadre des compositions tinctoriales de l'invention pour les bases d'oxydation de formule (I), les bases d'oxydation additionnelles et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

Les sels d'addition avec une base utilisables dans le cadre de l'invention sont par exemple choisis parmi les sels d'addition avec la soude, la potasse, l'ammoniaque, les amines ou les alcanolamines.

La composition tinctoriale conforme à l'invention peut en outre contenir un ou plusieurs colorants directs pouvant notamment être choisis parmi les colorants nitrés de la série benzénique, les colorants directs cationiques, les colorants directs azoïques, les colorants directs méthiniques.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non, modifiées, des agents filmogènes, des céramides, des agents conservateurs, des agents opacifiants.

Ces adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III) suivante : 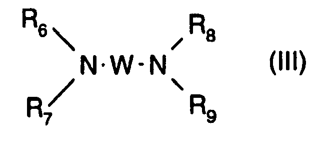 dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition de l'invention.

Selon ce procédé, on applique sur les fibres la composition selon la présente invention, la couleur étant révélée à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment pour la composition de l'invention.

Le pH de la composition oxydante renfermant l'agent oxydant est tel qu'après mélange avec la composition tinctoriale, le pH de la composition résultante appliquée sur les fibres kératiniques varie entre 3 et 12 environ, et préférentiellement entre 5 et 11. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

Un autre objet de l'invention est un dispositif à plusieurs compartiments ou "kit" de teinture dans lequel un premier compartiment renferme la composition pour la teinture définie ci-dessus et un deuxième compartiment renferme la composition oxydante. Ce dispositif peut être équipé d'un moyen permettant de délivrer sur les cheveux le mélange souhaité, tel que les dispositifs décrits dans le brevet FR-2 586 913 au nom de la demanderesse.

Enfin l'invention a également pour objet le produit coloré susceptible d'être obtenu par condensation oxydative de la composition de la présente invention. Cette composition comprend au moins une base d'oxydation de formule (I) telle que définie ci-dessus en présence d'au moins agent oxydant tel que défini précédemment et éventuellement en présence d'au moins un coupleur et/ou d'au moins une base d'oxydation additionnelle.

Ces produits colorés peuvent notamment se présenter sous la forme de pigments et être utilisés à titre de colorant direct pour la teinture directe des cheveux ou bien encore être incorporés dans des produits cosmétiques tels que par exemple dans des produits de maquillage.

La présente invention a enfin pour objet les nouveaux dérivés de paraphénylènediamine de formule (I) à l'exception du 4-(4-méthyl-[1,4] diazepan-1-yl)-phénylamine, 3-cyano 4-(4-t-butoxycarbonyl 1,4-cyclodiazacycloheptane)aniline, 4-(4-t-butoxycarbonyl 1,4-diazacycloheptane)aniline; 3-fluoro-4-(N-tert-butoxycarbonyl homo-piperazinyl)aniline (WO-A-9858926, WO-A-0047558, Bioorg. Med. Chem. 2004, 12(9), 2179-92, WO-A-0206278).

Les exemples qui suivent servent à illustrer l'invention sans toutefois présenter un caractère limitatif.

### EXEMPLES

### Exemple 1 : Synthèse du 4-(4-Méthyl-[1,4]diazepan-1-yl)-phénylamine dichlorhydrate (2)

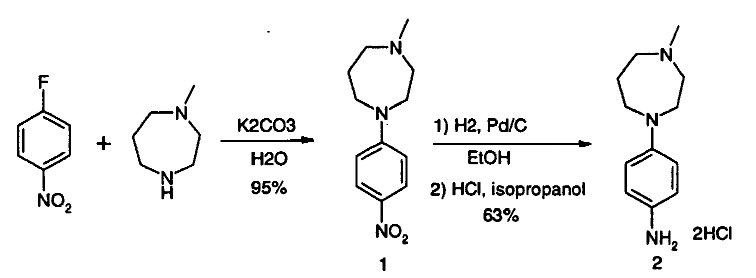

### Etape 1 : synthèse du 1-Méthyl-4-(4-nitro-phényl)-[1,4]diazerpane (1):

Le fluoronitrobenzene (10,3 g, 0,073 mol.) est mis en solution dans l'eau puis le carbonate de potassium (12,1 g, 0,0876 mol.) est ajouté. Le 1-méthyl [1,4] diazépane (10g, 0,0876 mol.) est introduit puis le milieu réactionnel est chauffé à reflux (≅100°c) pendant 5 heures. On refroidit le milieu. Il se forme un précipité que l'on filtre et que l'on lave à l'eau. On récupère un solide jaune qui est séché à 45°C dans une étuve à vide.(16,21 g, Rdt = 94,5%).

### Données spectroscopiques :

■ 1 H-RMN (DMSO d6, 200 MHz) : 1.63-1.75 (*m*, 2H), 2.06 (s, 3H), 2.22-2.33 (*m*, 2H), 2.40-2.45 (*m*, 2H), 3.34-3.51 (*m*, 4H), 6.58-6.66 (*m*, 2H), 7.79-7.88 (*m*, 2H)

### Etape 2 : synthèse du 4-(4-Méthyl-[1,4]diazepan-1-yl)-phénylamine, dichlorhydrate (2) :

Dans un réacteur à hydrogéner en inox, on dissout partiellement 6 g de 1-Méthyl-4-(4-nitro-phényl)-[1,4]diazépane (1) (25,5 mmol) dans 300 ml d'éthanol. On ajoute 2,1 g de Pd/C à 5 % (50% humide), on ferme le réacteur et le purge à l'azote 3 fois sous agitation (1800 tours/min). On introduit ensuite l'hydrogène sous une pression de 12 bars à température ambiante pendant 4 heures. Le réacteur est alors purgé à l'azote et le milieu réactionnel est filtré sous atmosphère d'azote et le filtrat est récupéré immédiatement dans une solution contenant 9.6 ml d'acide chlorhydrique à 37% et 50 ml d'isopropanol. Le filtrat est ensuite concentré jusqu'à l'obtention d'un précipité. Le solide est filtré, lavé à l'isopropanol puis séché sous vide en présence de potasse. On obtient, ainsi 4,5 g (63%) de 4-(4-Méthyl-[1,4]diazepan-1-yl)-phénylamine, dichlorhydrate (2) sous forme d'un solide blanc.

### Données spectroscopiques :

■ 1 H-RMN (D20 d6, 400 MHz) : 2.16 (*m*, 1 H), 2.35 (*m*, 1 H), 2.77 (*s*, 3H), 3.08-3.15 (*m*, 2H), 3.38-3.46 (*m*, 4H), 3.73-3.80 (*m*, 2H), 6.82 (*d*, *J*=9.2 Hz , 2H), 7.21 (*d*, J=9.2Hz, 2H)
■ 13C-RMN (MeOD, 100 MHz) : 23.3, 43.05, 43.44, 46.85, 54.90, 55.88
■ Spectre de masse : spectre en accord avec la structure

### Exemple 2 : Synthèse du 4-[1,4]Diazepan-1-yl-2-méthyl-phénylamine, dichlorhydrate (3)

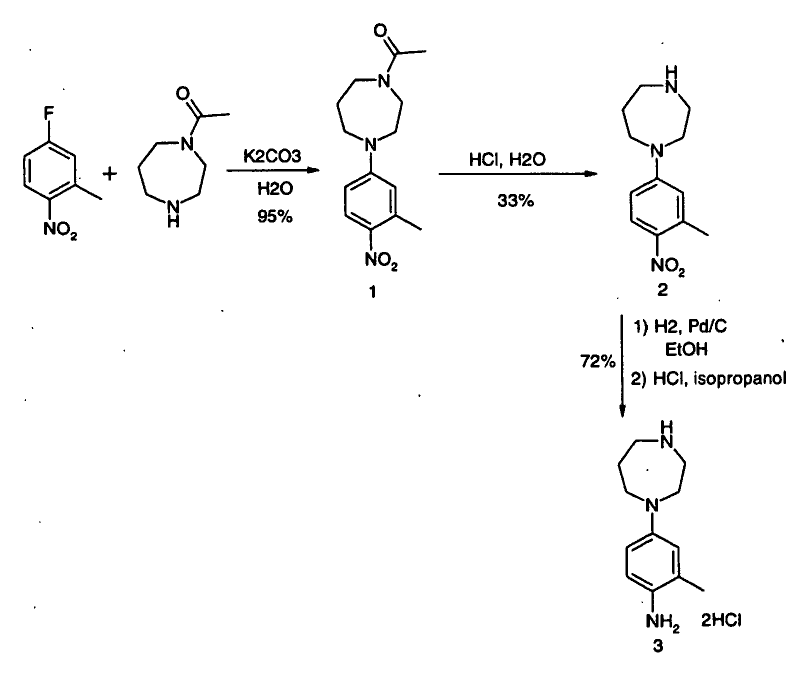

### Etape 1 : synthèse du 1-(3-Méthyl-4-nitro-phényl)-[1,4]diazepane (1):

La fluoronitrobenzène (9,1 g, 0,0586 mol.) est mis en solution dans l'eau (50 ml) puis on ajoute le carbonate de potassium (9,7 g, 0,0703 mol.) et la 1-acétyl)-[1,4]diazepane (10 g, 0,0703 mol.). On chauffe à 95°C pendant 6 heures, on obtient une solution orange. On refroidit ensuite à température ambiante, il y a alors apparition d'une pâte collante orange. On enlève le maximum d'eau du milieu réactionnel. Puis on ajoute de l'isopropanol à la pâte jusqu'à précipitation d'un solide jaune. On filtre et on obtient, après séchage dans une étuve à vide à 45°C, une poudre jaune qui correspond au 1-(3-Méthyl-4-nitro-phényl)-[1,4]diazépane (1). (8,7g ; Rdt = 53,6 %)

### Données spectroscopiques:

■ 1 H-RMN (DMSO d6, 200 MHz) : 1.57-1.81 (m, 5H), 2.39 (s, 3H), 3.13-3.25 (m, 4H), 3.38-3.52 (m, 5H), 3.60-3.65 (m, 1 H), 6.56-6.63 (m, 2H), 7.79-7.85 (m, 1 H)

### Etape 2: synthèse du 1-(3-Méthyl-4-nitro-phényl)-[1,4]diazépane (2):

Le 1-(3-Méthyl-4-nitro-phényl)-[1,4]diazépane 1 (8 g, 0,0289 mol.) est mis en suspension dans une solution d'HCl à 37% (29 ml) dans de l'eau (145 ml). On chauffe à reflux (100° c) pendant 30 heures. On laisse refroidir, on obtient alors un précipité marron. On ajoute environ 30 ml de soude à 35 % jusqu'a pH= 7. Le milieu réactionnel est ensuite concentré jusqu'à l'apparition d'un solide. Après filtration, ce solide est recristallisé dans un mélange isopropanol/méthanol. On obtient ainsi une poudre marron correspondant au 1-(3-Méthyl-4-nitro-phényl)-[1,4]diazépane (2).(2,4 g, Rdt = 33 %)

### Données spectroscopiques :

■ 1 H-RMN (DMSO d6, 200 MHz) : 2.18 (*m*, 2H), 2.65 (*s*, 3H), 3.12-3.14 (*m*, 2H), 3.25 (*m*, 2H), 3.70-3.76 (*m*, 2H), 3.92 (*m*, 2H), 6.84 (*m*, 2H), 8.02-8.12 (*m*, 1 H), 9.03-9.33 (m, 1 H)

### Etape 3 : synthèse du 4-[1,4]Diazépan-1-yl-2-méthyl-phénylamine, dichlorhydrate (3):

Dans un réacteur à hydrogéner en inox, on dissout 2,34 g de 1-(3-Méthyl-4-nitro-phényl)-[1,4]diazépane 2 (9,96 mmol) dans 150 ml d'éthanol. On ajoute 1,2 g de Pd/C à 5 % (50% humide), on ferme le réacteur et le purge à l'azote 3 fois sous agitation (1800 tours/min). On introduit ensuite l'hydrogène sous une pression de 9 bars à température ambiante pendant 3 heures. Le réacteur est alors purgé à l'azote et le milieu réactionnel est filtré sous atmosphère d'azote et le filtrat est récupéré immédiatement dans une solution contenant 3,7 ml d'acide chlorhydrique à 37% et 16 ml d'isopropanol. Le filtrat est ensuite concentré jusqu'à l'obtention d'un précipité. Le solide est filtré, lavé à l'isopropanol puis séché sous vide en présence de potasse. On obtient ainsi 2 g (72%) de 4-[1,4]Diazépan-1-yl-2-méthyl-phénylamine, dichlorhydrate (3) sous forme d'un solide blanc.

### Données spectroscopiques :

■ 1 H-RMN (D2O d6, 200 MHz) : 1.92-1.99 (m, 2H), 2.09 (m, 3H), 3.03-3.09 (m, 2H), 3.18-3.23 (m, 2H), 3.36-3.40 (m, 2H), 3.56-3.61 (m, 2H), 6.59-6.60 (m, 2H), 7.00 (d, J=8.7 Hz, 1 H)
■ Spectre de masse : spectre conforme

### Exemple 3 : Synthèse du 4-[4-(3-Pyrrolidin-1-yl-propyl)-[1,4]diazepan-1-yl]-phénylamine, trichlorhydrate (2)

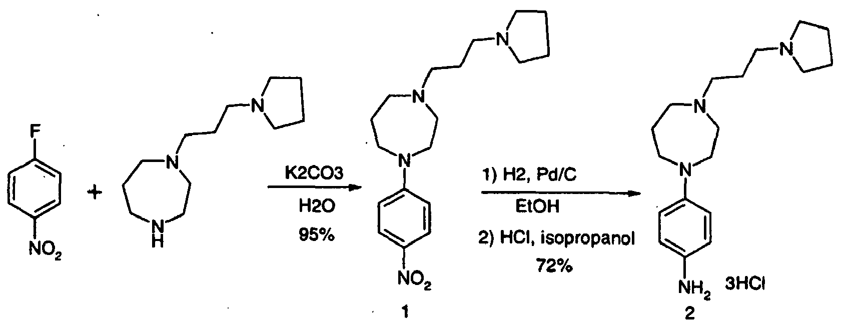

### Etape 1 : synthèse du 1-(4-Nitro-phényl)-4-(3-pyrrolidin-1-yl-propyl)-[1,4]diazepane (1):

Le fluoronitrobenzène (5,56 g, 39,43 mmole) est mis dans l'eau, puis on ajoute le carbonate de potassium (6,55 g, 47,31 mmoles) et la 1-(3-Pyrrolidiropropyl)-homopiperazine (10 g, 47,31 mmoles). On chauffe à 85° pendant 4 heures puis on refroidit à température ambiante. On filtre et on récupère après séchage dans une étuve à vide à 45°C un solide jaune (12,5 g, rdt = 95,5,%).

### Données spectroscopiques :

- 1 H-RMN (DMSO d6, 200 MHz) : 1.55-1.71 (*m*, 6H), 1.90-1.96 (*m*, 2H), 2.32-2.61 (*m*, 10H), 2.75-2.80 (*m*, 2H), 3.63-3.73 (*m*, 4H), 6.89 (*d*, J=9.4 Hz, 2H), 8.09 (*d*, J=9.4 Hz, 2H)

### Etape 2 : synthèse du, 4-[4-(3-Pyrrolidin-1-yl-propyl)-[1,4]diazépan-1-yl]-phénylamine, trichlorhydrate (2):

Dans un réacteur à hydrogéner en inox, on dissout 6,6 g de 1-(4-Nitro-phényl)-4-(3-pyrrolidin-1-yl-propyl)-[1,4]diazepane (1) (19,9 mmol) dans 330 ml d'éthanol. On ajoute 2,3 g de Pd/C à 5 % (50% humide), on ferme le réacteur et le purge à l'azote 3 fois sous agitation (1800 tours/min). On introduit ensuite l'hydrogène sous une pression de 10 bars à température ambiante pendant 3 heures. Le réacteur est alors purgé à l'azote et le milieu réactionnel est filtré sous atmosphère d'azote et le filtrat est récupéré immédiatement dans une solution contenant 9,1 ml d'acide chlorhydrique à 37% et 40 ml d'isopropanol. Le filtrat est ensuite concentré jusqu'à l'obtention d'un précipité. Le solide est filtré, lavé à l'isopropanol puis séché sous vide en présence de potasse. On obtient ainsi 5,9 g (72%) de 4-[4-(3-Pyrrolidin-1-yl-propyl)-[1,4]diazepan-1-yl]-phénylamine, trichlorhydrate (2) sous forme d'un solide blanc.

### Données spectroscopiques :

■ 1 H-RMN (D2O d6, 500 MHz) : 2.02-2.04 (*m*, 2H), 2.18 (*m*, 2H), 2.18-2.31 (*m*, 4H), 3.12-3.14 (*m*, 2H), 3.30-3.35 (*m*, 4H), 3.57-3.59 (*m*, 2H), 3.71-3.72 (*m*, 2H), 3.87 (*m*, 2H) 6.94 (*d*, J=8.9 Hz, 2H), 7.32 (*d*, J=8.9 Hz, 2H)
■ Spectre de masse : spectre en accord avec la structure

### Exemple 4 : Synthèse du 4-[1,4]Diazepan-1-yl-phénylamine, dichlorhydrate (2)

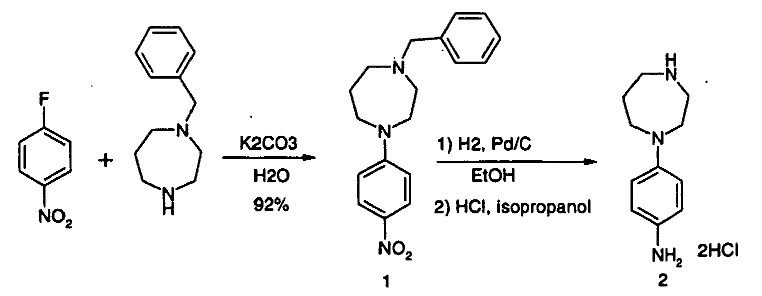

### Etape 1 : synthèse du 1-Benzyl-4-(4-nitro-phényl)-[1,4]diazepane (1):

On met le fluoronitrobenzène (3,09 g, 21,9 mmoles) dans l'eau (14 ml), puis on ajoute le carbonate de potassium (3,64 g, 26,28 mmoles) et le 1-benzyl homopiperazine (5 g, 26,28 mmoles). On fait chauffer à 90°c pendant 4h20 et ensuite on refroidit à température ambiante. On filtre le précipité formé, on obtient un solide jaune. On récupère 6,30g (92.5%) de produit.

### Données spectroscopiques :

■ 1 H-RMN (DMSO d6, 500 MHz) : 2.08-2.16 (*m*, 2H), 2.74-2.80 (*m*, 2H), 2.92-2.97 (*m*, 2H), 3.85-3.92 (*m*, 6H), 7.07 (*d*, J=9.5 Hz, 2H), 7.44-7.59 (*m*, 5H), 8.27 (*d*, J=9.5 Hz, 2H)

### Etape 2 : synthèse du 4-[1,4]Diazépan-1-yl-phénylamine, dichlorhydrate (2):

Dans un réacteur à hydrogéner en inox, on dissout 6,3 g de 1-Benzyl-4-(4-nitro-phényl)-[1,4]diazepane 1 (22,26 mmol) dans 170 ml d'éthanol. On ajoute 2,3 g de Pd/C à 5 % (50% humide), on ferme le réacteur et le purge à l'azote 3 fois sous agitation (1800 tours/min). On introduit ensuite l'hydrogène sous une pression de 11 bars, on chauffe jusqu'à une température de 60°C puis on laisse refroidir pendant 3,5 heures. Le réacteur est alors purgé à l'azote et le milieu réactionnel est filtré sous atmosphère d'azote et le filtrat est récupéré immédiatement dans une solution contenant 7,6 ml d'acide chlorhydrique à 37% et 30 ml d'isopropanol. Le filtrat est ensuite concentré jusqu'à l'obtention d'un précipité. Le solide est filtré, lavé à l'isopropanol puis séché sous vide en présence de potasse. On obtient ainsi 4,6 g de 4-[1,4]Diazepan-1-yl-phénylamine, dichlorhydrate (2) sous forme d'un solide blanc.

### Données spectroscopiques :

■ 1 H-RMN (D20, 500 MHz) : 2.23-2.28 (*m*, 2H), 3.36-3.39 (*m*, 2H), 3.52-3.54 (*m*, 2H), 3.65-3.69 (*m*, 2H), 3.89-3.91 (*m*, 2H), 7.07 (*m*, 2H), 7.37 (*m*, 2H)
■ Spectre de masse : spectre en accord avec la structure

### Exemple 5 : Synthèse du 2-[4-(4-Amino-phényl)-[1,4]diazepan-1-yl]-ethanol, dichlorhydrate (2)

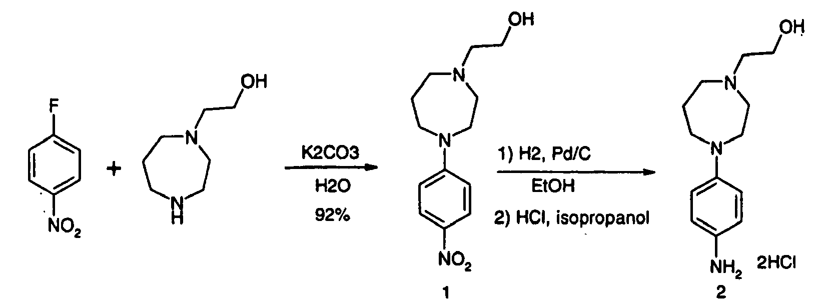

### Etape 1 : :synthèse du 2-[4-(4-Nitro-phényl)-[1,4]diazépan-1-yl]-éthanol (1):

La fluoronitrobenzène (2,03 g, 14,4 mmoles) est mis dans l'eau (10 ml), puis on ajoute le carbonate de potassium (2,4 g, 17,3 mmoles) et la 1-(2-hydroxyethyl) homopiperazine. On chauffe à 95°c pendant 4 heures, puis on refroidit à température ambiante et on filtre. On récupère une poudre orange foncée que l'on met à l'étuve (3,5g, Rdt =91,7%).

### Données spectroscopiques :

■ 1 H-RMN (DMSO d6, 200 MHz) : 1.66-1.78 (*m*, 2H), 2.37-2.48 (*m*, 6H), 2.63-2.68 (*m*, 2H), 3.29-3.35 (*m*, 2H), 3.44-3.69 (*m*, 4H), 4.26 (*m*, 1 H), 6.66-6.74 (*m*, 2H), 7.86-7.95 (*m*, 2H)

### Etape 2 : synthèse du 2-[4-(4-Amino-phényl)-[1,4]diazepan-1-yl]-éthanol, dichlorhydrate (2) :

Dans un réacteur à hydrogéner en inox, on dissout 6,9 g de 2-[4-(4-Nitro-phényl)-[1,4]diazepan-1-yl]-ethanol (26,04 mmol) dans 350 ml d'éthanol. On ajoute 2,5 g de Pd/C à 5 % (50% humide), on ferme le réacteur et le purge à l'azote 3 fois sous agitation (1800 tours/min). On introduit ensuite l'hydrogène sous une pression de 9 bars pendant 4,5 heures. Le réacteur est alors purgé à l'azote et le milieu réactionnel est filtré sous atmosphère d'azote et le filtrat est récupéré immédiatement dans une solution contenant 9,8 ml d'acide chlorhydrique à 37% et 40 ml d'isopropanol. Le filtrat est ensuite concentré jusqu'à l'obtention d'un précipité. Le solide est filtré, lavé à l'isopropanol puis séché sous vide en présence de potasse. On obtient ainsi 7,8 g de 2-[4-(4-Amino-phényl)-[1,4]diazepan-1-yl]-éthanol, dichlorhydrate (2) sous forme d'un solide blanc.

### Données spectroscopiques :

■ 1 H-RMN (D2O d6, 500 MHz) : 2.17-2.18 (*m*, 1H), 2.49-2.50 (*m*, 1H), 3.10-3.21 (*m,* 4H), 3.41-3.55 (*m*, 4H), 3.78-3.81 (*m*, 4H), 6.83 (*m*, 2H), 7.22 (*m*, 2H)
■ Spectre de masse : spectre en accord avec la structure
■ Analyse élémentaire

| | %C | %H | %N | %O | %Cl |
|---|---|---|---|---|---|
| Théorie | 50.66 | 7.52 | 13.63 | 5.19 | 23 |
| Trouvé | 50.17 | 7.53 | 13.59 | 5.49 | 22.99 |

### Exemples de compositions

On a préparé les compositions suivantes (teneur des bases et coupleurs en mole)

| **Exemples** | **6** | **7** | **8** | **9** | **10** | **11** | **12** |
|---|---|---|---|---|---|---|---|
| 4-[1,4]Diazepan-1-yl-2-méthyl- phénylamine, dichlorhydrate | 3x10⁻³ | 3x10⁻³ | 3x10⁻³ | 3x10⁻³ | - | - | - |
| 4-[4-(3-Pyrrolidin-1-yl-propyl)- [1,4]diazepan-1-yl]-phénylamine, trichlorhydrate | - | - | - | - | 3x10⁻³ | 3x10⁻³ | 3x10⁻³ |
| Dihydroxy 1,3 benzène | - | 3x10⁻³ | - | - | 3x10⁻³ | - | - |
| 1-β-hydroxyéthyloxy-2,4- diaminobenzène, 2HCl | - | - | 3x0⁻³ | - | - | 3x10⁻³ | - |
| 2-méthyl-5-amino phénol | - | - | - | 3x10⁻³ | - | - | 3x10⁻³ |
| Support de teinture | (*) | (*) | (*) | (*) | (*) | (*) | (*) |
| Eau déminéralisée q.s.p. | 100 g | 100 g | 100 g | 100 g | 100 g | 100 g | 100 g |

| **Exemples** | **13** | **14** | **15** | **16** | **17** | **18** | **19** | **20** |
|---|---|---|---|---|---|---|---|---|
| 4-[1,4]Diazepan-1-yl- phénylamine, dichlorhydrate | 3x10⁻³ | 3x10⁻³ | 3x10⁻³ | 3x10⁻³ | - | - | - | - |
| 2-[4-(4-Amino-phényl)-[1,4]diazepan-1-yl]-ethanol, dichlorhydrate | - | - | - | - | 3x10⁻³ | 3x10⁻³ | 3x10⁻³ | 3x10⁻³ |
| Dihydroxy 1,3 benzène | - | 3x10⁻³ | - | - | - | 3x10⁻³ | - | - |
| 1-β-hydroxyéthyloxy-2,4-diaminobenzène, 2HCl | - | - | 3x10⁻³ | - | - | - | 3x10⁻³ | - |
| 2-méthyl-5-amino phénol | - | - | - | 3x10⁻³ | - | - | - | 3x10⁻³ |
| Support de teinture | (*) | (*) | (*) | (*) | (*) | (*) | (*) | (*) |
| Eau déminéralisée q.s.p. | 100 g | 100 g | 100 g | 100 g | 100 g | 100 g | 100 g | 100 g |

### Support de teinture (*)

- Alcool oléique polyglycérolé à 2 moles de glycérol 4,0 g
- Alcool oléique polyglycérolé à 4 moles de glycérol à 78 % de matières actives (M.A.) 5,69 g M.A.
- Acide oléique 3,0 g
- Amine oléique à 2 moles d'oxyde d'éthylène vendue sous la dénomination commerciale ETHOMEEN 012 par la société AKZO 7,0g
- Laurylamino succinamate de diéthylaminopropyle, sel de sodium à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium à en solution aqueuse à 35 % de M.A. 0,455 g M.A.
- Acétate d'ammonium 0,8 g
- Antioxydant, séquestrant q.s.
- Parfum, conservateur q.s.
- Ammoniaque à 20 % de NH₃ 10,0 g

Au moment de l'emploi, chaque composition est mélangée avec un poids égal d'eau oxygénée à 20 volumes (6% en poids).

Chaque mélange obtenu est appliqué sur des mèches de cheveux gris à 90 % de blancs naturels ou permanentés. Après 30 min de pose, les mèches sont rinçées, lavées avec un shampooing standard, rinçées à nouveau puis séchées.

La couleur des mèches a été évaluée dans le système L*a*b*, au moyen d'un spectrophotomètre CM 2002 MINOLTA.

Dans l'espace L*a*b*, la clarté est indiquée par la valeur L* sur une échelle de 0 à 100 alors que les coordonnées chromatiques sont exprimées par a* et b* qui indiquent deux axes de couleur, a* l'axe rouge-vert et b* l'axe jaune-bleu.

Selon ce système, plus la valeur de L est élevée, plus la couleur est claire et peu intense. Inversement, plus la valeur de L est faible, plus la couleur est foncée ou très intense.

Les résultats sont reportés dans le tableau 1 ci dessous

**TABLEAU 1**

| **Exemples** | **Nature des mèches** | **L*** | **a*** | **b*** |
|---|---|---|---|---|
| **Réf.1** | naturelle | 61,06 | 0,72 | 11,74 |
| **Réf.2** | permanentée | 59,7 | 0,24 | 9,10 |
| **6** | naturelle | 50,38 | 3,45 | 7,46 |
| **7** | naturelle | 38,86 | 5,30 | 5,97 |
| **8** | naturelle | 28,26 | -0,41 | -12,60 |
| **9** | naturelle | 32,71 | 10,85 | -7,57 |
| **10** | naturelle | 32,01 | 5,87 | 3,38 |
| **11** | naturelle | 27,47 | -0,98 | -11,14 |
| **12** | naturelle | 33,39 | 8,39 | -7,77 |
| **13** | naturelle | 55,66 | 4,21 | 10,12 |
| **14** | naturelle | 49,12 | 3,93 | 9,59 |
| **15** | naturelle | 39,85 | -5,27 | -0,29 |
| **16** | naturelle | 45,92 | -0,57 | -3,41 |
| **17** | naturelle | 39,97 | 1,53 | 5,77 |
| | permanentée | 33,47 | 2,56 | 5,98 |
| **18** | naturelle | 30,79 | 5,35 | 4,86 |
| | permanentée | 25,74 | 4,60 | 3,94 |
| **19** | naturelle | 22,04 | 2,19 | -10,60 |
| | permanentée | 20,04 | 1,67 | -5,66 |
| **20** | naturelle | 25,15 | 9,51 | -5,83 |
| | permanentée | 20,50 | 6,67 | -4,54 |

## Revendications

1. Composition pour la teinture par oxydation des fibres kératiniques comprenant, dans un milieu approprié, une base d'oxydation de formule (I) suivante ou les sels d'addition correspondants 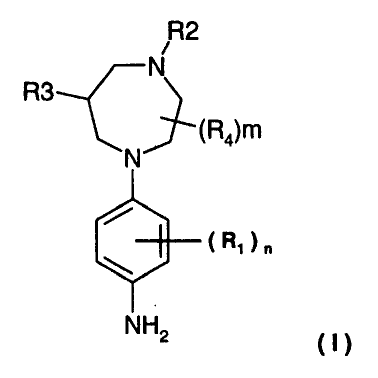 dans laquelle :
• **R₁** représente
- un atome d'halogène, de préférence chlore ou brome ;
- une chaîne hydrocarbonée en C₁-C₆, saturée ou pouvant contenir une ou plusieurs liaisons doubles et/ou une ou plusieurs liaisons triples, linéaire ou ramifiée pouvant former un cycle ayant de 3 à 6 chaînons, un ou plusieurs atomes de carbone pouvant être remplacés par un atome d'oxygène, d'azote ou de soufre, par un groupement SO₂, ou lorsque le carbone est terminal par un atome d'halogène, de préférence un atome de chlore ou de brome ; ledit radical R₁ ne comportant pas de liaison peroxyde, ni de radicaux diazo, nitro ou nitroso ;
• **n** est compris entre 0 et 4 inclus, étant entendu que lorsque n est supérieur ou égal à 2 alors les radicaux R₁ peuvent être identiques ou différents,
• **R₂** représente
- un atome d'hydrogène ;
- un radical alkyle pouvant être insaturé, non substitué ou substitué par un ou plusieurs radicaux carboxylique, alkylcarbonyle, alcoxycarbonyle, carbamoyle, mono- ou di-alkylcarbamoyle, hétérocyclique azoté, oxygéné et/ou soufré, saturé et/ou insaturé à 4, 5, 6 ou 7 atomes ;
- un radical alkyle pouvant être insaturé, substitué en position 2 ou plus par un ou plusieurs radicaux hydroxy, alcoxy, amino, mono- ou di-alkylamino, thiol ou halogène;
- un radical alkylcarbonyle ;
- un radical alkoxycarbonyle ;
- un radical mono- ou di-alkylcarbamoyle ;
- un radical carbamoyle ;
- un radical R₆R₇N-C=NR₅- où R₅, R₆ et R₇ représentent un hydrogène, un radical alkyle en C₁-C₄ ou un radical hydroxyalkyle, de préférence R₅ est l'hydrogène et R₆ et R₇ sont choisis parmi l'hydrogène ou un méthyle,
• **R₃** représente
- un atome d'hydrogène,
- un radical alkyle pouvant être insaturé ;
- un radical hydroxy,
- un radical hydroxyalkyle ;
- un radical alcoxy ;
- un radical alcoxyalkyle ;
- un radical alkylcarbonyle ;
- un radical hydroxyalcoxyalkyle ;
- un radical amino,
- un radical moalkylamino ou dialkylamino ;
- un radical aminoalkyle, l'amine pouvant être mono ou disubstitué par un radical alkyle, acétyle ou hydroxyalkyle ;
- un radical hydroxy- et amino- alkyle ;
- un radical carboxyle ;
- un radical carboxyalkyle ;
- un radical carbamoyle ;
- un radical carbamoylalkyle ;
- un radical alkoxycarbonyle ;
- un radical mono- ou dialkyl- aminocarbonyle ;
• **R₄** représente
- un radical alkyle pouvant être insaturé ;
- un radical hydroxyalkyle ;
- un radical alcoxyalkyle ;
- un radical alkylcarbonyle ;
- un radical hydroxyalcoxyalkyle ;
- un radical aminoalkyle, l'amine pouvant être mono ou disubstitué par un radical alkyle, acétyle, hydroxyalkyle ;
- un radical hydroxy- et amino- alkyle ;
- un radical carboxyle ;
- un radical carboxyalkyle ;
- un radical carbamoyle ;
- un radical carbamoylalkyle ;
- un radical alkoxycarbonyle ;
- un radical mono- ou dialkyl- aminocarbonyle ;
• m est compris entre 0 et 4 inclus, étant entendu que lorsque m est supérieur ou égal à 2 alors les radicaux R₄ peuvent être identiques ou différents.

2. Composition selon la revendication 1 dans laquelle R₁ est un radical alkyle, hydroxyalkyle ou aminoalkyle, alcoxy, hydroxyalcoxy.

3. Composition selon la revendication 1 ou 2 dans R₁ est choisi parmi un radical méthyle, hydroxyméthyle, 2-hydroxyéthyle, 1,2-dihydroxyéthyle, méthoxy, 2-hydroxyéthoxy.

4. Composition selon l'une quelconque des revendications précédentes dans laquelle n est égal à 0 ou 1.

5. Composition selon l'une quelconque des revendications précédentes dans laquelle R₂ est choisi parmi l'hydrogène, un radical alkyle, un radical alkyle substitué par un hétérocyclique azoté et/ou oxygéné, saturé ou insaturé, à 4, 5 ou 6 atomes, un radical alcoxycarbonyle, un radical alkyle substitué en position 2 ou plus par un ou plusieurs radicaux hydroxy.

6. Composition selon l'une quelconque des revendications précédentes dans laquelle R₂ est le radical 2-hydroxyéthyl, le radical 3 (1-pyrrolidinyl)propyle, le radical méthyle, le radical acétyle, l'hydrogène.

7. Composition selon l'une quelconque des revendications précédentes dans laquelle R₃ est choisi parmi l'hydrogène, un radical alkyle, un radical alkyle substitué par un ou plusieurs hydroxy, un radical alkyle substitué par un ou plusieurs amino, un radical carboxyle.

8. Composition selon l'une quelconque des revendications précédentes dans laquelle R₃ est choisi parmi le radical hydrogène, le radical hydroxyle, le radical carboxyle, le radical amino, le radical hydroxyméthyle, le radical aminométhyle.

9. Composition selon l'une quelconque des revendications précédentes dans laquelle R₄ est choisi parmi l'hydrogène, un radical alkyle, un radical alkyle substitué par un ou plusieurs hydroxy, un radical alkyle substitué par un ou plusieurs amino, un radical carboxyle.

10. Composition selon l'une quelconque des revendications précédentes dans laquelle R₄ est choisi parmi l'hydrogène.

11. Composition selon l'une quelconque des revendications précédentes dans laquelle la base d'oxydation de formule (I) est choisie parmi
4-(4-Méthyl-[1,4]diazepan-1-yl)-phénylamine
4-[1,4]Diazepan-1-yl-phénylamine
4-[4-(3-Pyrrolidin-1-yl-propyl)-[1,4]diazepan-1-yl]-phénylamine
2-[4-(4-Amino-phényl)-[1,4]diazepan-1-yl]-éthanol
1 -[4-(4-Amino-phényl)-[1,4]diazepan-1-yl]-éthanone
4-(4-Amino-phényl)-[1,4]diazepane-1-carboxamidine
4-(4-Amino-phényl)-N,N-diméthyl-[1,4]diazepane-1-carboxamidine
1-(4-Amino-phényl)-4-méthyl-[1,4]diazepan-6-ol
1-(4-Amino-phényl)-4-méthyl-[1,4]diazepan-6-ylamine
1 -(4-Amino-phényl)-4-(3-pyrrolidin-1-yl-propyl)-[1,4]diazepan-6-ol
1-(4-Amino-phényl)-4-(3-pyrrolidin-1-yl-propyl)-[1,4]diazepan-6-ylamine
1-(4-Amino-phényl)-4-(2-hydroxy-ethyl)-[1,4]diazepan-6-ol
2-[6-Amino-4-(4-amino-phényl)-[1,4]diazepan-1-yl]-éthanol
2-[4-(4-Amino-phényl)-6-hydroxyméthyl-[1,4]diazepan-1-yl]-éthanol
2-Méthyl-4-(4-méthyl-[1 ,4]diazepan-1-yl)-phénylamine
4-[1,4]Diazepan-1-yl-2-méthyl-phénylamine
2-Méthyl-4-[4-(3-pyrrolidin-1-yl-propyl)-[1,4]diazepan-1-yl]-phénylamine
2-[4-(4-Amino-3-méthyl-phényl)-[1,4]diazepan-1-yl]-ethanol
1-[4-(4-Amino-3-méthyl-phényl)-[1,4]diazepan-1-yl]-ethanone
4-(4-Amino-3-méthyl-phényl)-[1,4]diazepane-1-carboxamidine
4-(4-Amino-3-méthyl-phényl)-N,N-diméthyl-[1,4]diazepane-1-carboxamidine
1-(4-Amino-3-méthyl-phényl)-4-méthyl-[1,4]diazepan-6-ol
1-(4-Amino-3-méthyl-phényl)-4-méthyl-[1,4]diazepan-6-ylamine
1-(4-Amino-3-méthyl-phényl)-4-(3-pyrrolidin-1-yl-propyl)-[1,4]diazepan-6-ol
1-(4-Amino-3-méthyl-phényl)-4-(3-pyrrolidin-1-yl-propyl)-[1,4]diazepan-6-ylam ine
1-(4-Amino-3-méthyl-phényl)-4-(2-hydroxy-ethyl)-[1,4]diazepan-6-ol
2-[6-Amino-4-(4-amino-3-méthyl-phényl)-[1,4]diazepan-1-yl]-ethanol
2-[4-(4-Amino-3-méthyl-phényl)-6-hydroxyméthyl-[1,4]diazepan-1 -yl]-éthanol

12. Composition selon l'une quelconque des revendications précédentes dans laquelle la ou les bases d'oxydation de formule (I) sont présentes en quantité comprise 0,001 et 10 %, de préférence entre 0,005 et 6 %.

13. Composition selon l'une quelconque des revendications précédentes comprenant un ou plusieurs coupleurs choisis parmi les métaphénylènediamines, les méta-aminophénols, les métadiphénols, les coupleurs naphtaléniques et les coupleurs hétérocycliques et leurs sels d'addition.

14. Composition selon l'une quelconque des revendications comprenant une base d'oxydation additionnelle choisie parmi les paraphénylènediamines, les bis-phénylalkylènediamines, les para-aminophénols, les ortho-aminophénols, les bases hétérocycliques, et leurs sels d'addition.

15. Composition selon l'une quelconque des revendications précédentes comprenant de plus un colorant direct.

16. Procédé de teinture d'oxydation des fibres kératiniques **caractérisé en ce qu'**on applique sur les fibres au moins une composition telle que définie à l'une quelconque des revendications 1 à 15, et qu'on révèle la couleur à l'aide d'un agent oxydant.

17. Procédé selon la revendication 16 dans lequel l'agent oxydant est choisi parmi le peroxyde d'hydrogène, le peroxyde d'urée, les bromates de métaux alcalins, les persels, les peracides et les enzymes oxydases.

18. Procédé selon l'une des revendications 16 ou 17 dans lequel l'agent oxydant est mélangé au moment de l'emploi à la composition telle que définie selon l'une quelconque des revendications 1 à 15.

19. Procédé selon l'une quelconque des revendications 16 ou 17 dans lequel l'agent oxydant est appliqué sous forme de composition oxydante simultanément ou séquentiellement à la composition telle que définie selon l'une quelconque des revendications 1 à 15 sur les fibres.

20. Dispositif à plusieurs compartiments ou "kit" de teinture à plusieurs compartiments, dans lequel un premier compartiment contient une composition telle que définie à l'une quelconque des revendications 1 à 15 et un deuxième compartiment contient une composition oxydante.

21. Produit coloré susceptible d'être obtenu par condensation oxydative d'une composition telle que définie selon l'une quelconque des revendications 1 à 15.

22. Dérivé de paraphénylènediamine de formule (I) telle que définie dans l'une quelconque des revendications 1 à 11 à l'exception du 4-(4-méthyl-[1,4] diazepan-1-yl)-phénylamine, 3-cyano 4-(4-t-butoxycarbonyl 1,4-cyclodiazacycloheptane) aniline, 4-(4-tert-butoxycarbonyl 1,4-diazacycloheptane)aniline, 3-fluoro-4-(N-tert-butoxycarbonylhomopiperazinyl)aniline.

## Claims

1. Composition for the oxidation dyeing of keratin fibres, comprising, in a suitable medium, an oxidation base of formula (I) below, or the corresponding addition salts: 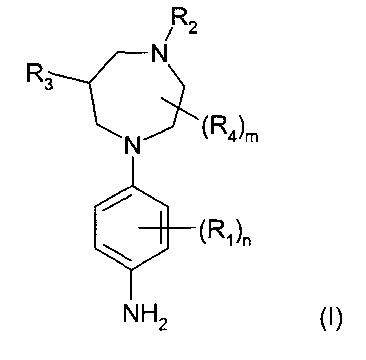 in which:
• **R₁** represents:
- a halogen atom, preferably chlorine or bromine;
- a linear or branched C₁-C₆ hydrocarbon-based chain, which is saturated or which may contain one or more double bonds and/or one or more triple bonds, possibly forming a 3- to 6-membered ring, one or more carbon atoms possibly being replaced with an oxygen, nitrogen or sulfur atom or with an SO₂ group, or, when the carbon is terminal, with a halogen atom, preferably a chlorine or bromine atom; the said radical R₁ comprising no peroxide bonds or diazo, nitro or nitroso radicals;
• **n** is between 0 and 4 inclusive, it being understood that when n is greater than or equal to 2, then the radicals R₁ may be identical or different;
• **R₂** represents:
- a hydrogen atom;
- an optionally unsaturated alkyl radical, which may be unsubstituted or substituted with one or more carboxyl, alkylcarbonyl, alkoxycarbonyl, carbamoyl, monoalkylcarbamoyl or dialkylcarbamoyl radicals or saturated or unsaturated nitrogen-, oxygen- and/or sulfur-bearing heterocyclic radicals containing 4, 5, 6 or 7 atoms;
- an optionally unsaturated alkyl radical, substituted in position 2 or more with one or more hydroxyl, alkoxy, amino, monoalkylamino, dialkylamino, thiol or halogen radicals;
- an alkylcarbonyl radical;
- an alkoxycarbonyl radical;
- a mono- or dialkylcarbamoyl radical;
- a carbamoyl radical;
- a radical R₆R₇N-C=NR₅- in which R₅, R₆ and R₇ represent a hydrogen, a C₁-C₄ alkyl radical or a hydroxyalkyl radical; preferably R₅ is hydrogen and R₆ and R₇ are chosen from hydrogen or a methyl;
• **R₃** represents:
- a hydrogen atom;
- an optionally unsaturated alkyl radical;
- a hydroxyl radical;
- a hydroxyalkyl radical;
- an alkoxy radical;
- an alkoxyalkyl radical;
- an alkylcarbonyl radical;
- a hydroxyalkoxyalkyl radical;
- an amino radical;
- a monoalkylamino or dialkylamino radical;
- an aminoalkyl radical, the amine possibly being monosubstituted or disubstituted with an alkyl, acetyl or hydroxyalkyl radical;
- a hydroxyalkyl or aminoalkyl radical;
- a carboxyl radical;
- a carboxyalkyl radical;
- a carbamoyl radical;
- a carbamoylalkyl radical;
- an alkoxycarbonyl radical;
- a monoalkyl- or dialkylaminocarbonyl radical;
• **R₄** represents:
- an optionally unsaturated alkyl radical;
- a hydroxyalkyl radical;
- an alkoxyalkyl radical;
- an alkylcarbonyl radical;
- a hydroxyalkoxyalkyl radical;
- an aminoalkyl radical, the amine possibly being monosubstituted or disubstituted with an alkyl, acetyl or hydroxyalkyl radical;
- a hydroxyalkyl or aminoalkyl radical;
- a carboxyl radical;
- a carboxyalkyl radical;
- a carbamoyl radical;
- a carbamoylalkyl radical;
- an alkoxycarbonyl radical;
- a monoalkyl- or dialkylaminocarbonyl radical;
• **m** is between 0 and 4 inclusive, it being understood that when m is greater than or equal to 2, then the radicals R₄ may be identical or different.

2. Composition according to Claim 1, in which R₁ is an alkyl, hydroxyalkyl, aminoalkyl, alkoxy or hydroxy-alkoxy radical.

3. Composition according to Claim 1 or 2, in which R₁ is chosen from methyl, hydroxymethyl, 2-hydroxyethyl, 1,2-dihydroxyethyl, methoxy and 2-hydroxyethoxy radicals.

4. Composition according to any one of the preceding claims, in which n is equal to 0 or 1.

5. Composition according to any one of the preceding claims, in which R₂ is chosen from hydrogen, an alkyl radical, an alkyl radical substituted with a saturated or unsaturated nitrogen- and/or oxygen-bearing heterocyclic radical containing 4, 5 or 6 atoms, an alkoxycarbonyl radical, and an alkyl radical substituted in position 2 or more with one or more hydroxyl radicals.

6. Composition according to any one of the preceding claims, in which R₂ is a 2-hydroxyethyl radical, a 3-(1-pyrrolidinyl)propyl radical, a methyl radical, an acetyl radical or hydrogen.

7. Composition according to any one of the preceding claims, in which R₃ is chosen from hydrogen, an alkyl radical, an alkyl radical substituted with one or more hydroxyl groups, an alkyl radical substituted with one or more amino groups, and a carboxyl radical.

8. Composition according to any one of the preceding claims, in which R₃ is chosen from a hydrogen atom, a hydroxyl radical, a carboxyl radical, an amino radical, a hydroxymethyl radical and an aminomethyl radical.

9. Composition according to any one of the preceding claims, in which R₄ is chosen from hydrogen, an alkyl radical, an alkyl radical substituted with one or more hydroxyl groups, an alkyl radical substituted with one or more amino groups, and a carboxyl radical.

10. Composition according to any one of the preceding claims, in which R₄ is chosen from hydrogen.

11. Composition according to any one of the preceding claims, in which the oxidation base of formula (I) is chosen from:
4-(4-methyl[1,4]diazepan-1-yl)phenylamine
4-[1,4]diazepan-1-ylphenylamine
4-[4-(3-pyrrolidin-1-ylpropyl)[1,4]diazepan-1-yl]-phenylamine
2-[4-(4-aminophenyl)[1,4]diazepan-1-yl]ethanol
1-[4-(4-aminophenyl)[1,4]diazepan-1-yl]ethanone
4-(4-aminophenyl)[1,4]diazepane-1-carboxamidine
4-(4-aminophenyl)-N,N-dimethyl[1,4]diazepane-1-carboxamidine
1-(4-aminophenyl)-4-methyl[1,4]diazepan-6-ol
1-(4-aminophenyl)-4-methyl[1,4]diazepan-6-ylamine
1-(4-aminophenyl)-4-(3-pyrrolidin-1-ylpropyl)[1,4]-diazepan-6-ol
1-(4-aminophenyl)-4-(3-pyrrolidin-1-ylpropyl)[1,4]-diazepan-6-ylamine
1-(4-aminophenyl)-4-(2-hydroxyethyl)[1,4]diazepan-6-ol
2-[6-amino-4-(4-aminophenyl)[1,4]diazepan-1-yl]ethanol
2-[4-(4-aminophenyl)-6-hydroxymethyl[1,4]diazepan-1-yl]ethanol
2-methyl-4-(4-methyl[1,4]diazepan-1-yl)phenylamine
4-[1,4]diazepan-1-yl-2-methylphenylamine
2-methyl-4-[4-(3-pyrrolidin-1-ylpropyl)[1,4]diazepan-1-yl]phenylamine
2-[4-(4-amino-3-methylphenyl)[1,4]diazepan-1-yl]ethanol
1-[4-(4-amino-3-methylphenyl)[1,4]diazepan-1-yl]-ethanone
4-(4-amino-3-methylphenyl)[1,4]diazepane-1-carboxamidine
4-(4-amino-3-methylphenyl)-N,N-dimethyl[1,4]diazepane-1-carboxamidine
1-(4-amino-3-methylphenyl)-4-methyl[1,4]diazepan-6-ol
1-(4-amino-3-methylphenyl)-4-methyl[1,4]diazepan-6-ylamine
1-(4-amino-3-methylphenyl)-4-(3-pyrrolidin-1-ylpropyl)-[1,4]diazepan-6-ol
1-(4-amino-3-methylphenyl)-4-(3-pyrrolidin-1-ylpropyl)-[1,4]diazepan-6-ylamine
1-(4-amino-3-methylphenyl)-4-(2-hydroxyethyl)[1,4]-diazepan-6-ol
2-[6-amino-4-(4-amino-3-methylphenyl)[1,4]diazepan-1-yl]ethanol
2-[4-(4-amino-3-methylphenyl)-6-hydroxymethyl[1,4]-diazepan-1-yl]ethanol.

12. Composition according to any one of the preceding claims, in which the oxidation base(s) of formula (I) is (are) present in an amount of between 0.001% and 10% and preferably between 0.005% and 6%.

13. Composition according to any one of the preceding claims, comprising one or more couplers chosen from meta-phenylenediamines, meta-aminophenols, meta-diphenols, naphthalene-based couplers and heterocyclic couplers, and the addition salts thereof.

14. Composition according to any one of the preceding claims, comprising an additional oxidation base chosen from para-phenylenediamines, bis(phenyl)alkylenediamines, para-aminophenols, ortho-aminophenols and heterocyclic bases, and the addition salts thereof.

15. Composition according to any one of the preceding claims, also comprising a direct dye.

16. Process for the oxidation dyeing of keratin fibres, **characterized in that** at least one composition as defined in any one of Claims 1 to 15 is applied to the fibres, and the colour is revealed using an oxidizing agent.

17. Process according to Claim 16, in which the oxidizing agent is chosen from hydrogen peroxide, urea peroxide, alkali metal bromates, persalts, peracids and oxidase enzymes.

18. Process according to either of Claims 16 and 17, in which the oxidizing agent is mixed at the time of use with the composition as defined according to any one of Claims 1 to 15.

19. Process according to either of Claims 16 and 17, in which the oxidizing agent is applied to the fibres in the form of an oxidizing composition, simultaneously with or sequentially to the composition as defined according to any one of Claims 1 to 15.

20. Multi-compartment device or multi-compartment dyeing "kit", in which a first compartment contains a composition as defined in any one of Claims 1 to 15 and a second compartment contains an oxidizing composition.

21. Coloured product that may be obtained via oxidative condensation of a composition as defined according to any one of Claims 1 to 15.

22. para-Phenylenediamine derivative of formula (I) as defined in any one of Claims 1 to 11, with the exception of 4-(4-methyl[1,4]diazepan-1-yl)phenylamine, 3-cyano-4-(4-t-butoxycarbonyl-1,4-cyclodiazacycloheptane)aniline, 4-(4-tert-butoxycarbonyl-1,4-diazacycloheptane)aniline and 3-fluoro-4-(N-tert-butoxycarbonyl-homopiperazinyl)aniline.

## Patentansprüche

1. Zusammensetzung zum oxidativen Färben von Keratinfasern, die in einem geeigneten Medium eine Oxidationsbase der folgenden Formel (I) oder die entsprechenden Additionssalze enthält: 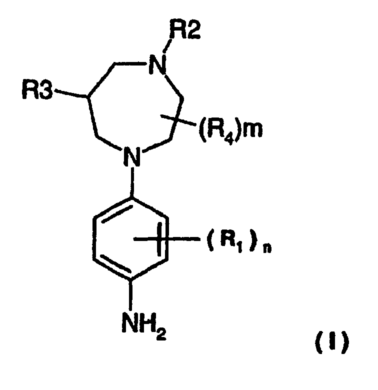 wobei in der Formel:
• **R₁** bedeutet
- ein Halogenatom, vorzugsweise Chlor oder Brom;
- eine Kohlenwasserstoffkette mit 1 bis 6 Kohlenstoffatomen, die gesättigt ist oder eine oder mehrere Doppelbindungen und/oder eine oder mehrere Dreifachbindungen enthalten kann und die geradkettig oder verzweigt vorliegt, wobei sie einen 3- bis 6-gliedrigen Ring bilden kann und wobei ein oder mehrere Kohlenstoffatome durch ein Sauerstoffatom, Stickstoffatom oder Schwefelatom oder durch eine Gruppe -SO₂ oder, wenn es sich um das endständige Kohlenstoffatom handelt, durch ein Halogenatom und vorzugsweise ein Chloratom oder Bromatom ersetzt sein können; wobei die Gruppe R₁ weder eine Peroxidbindung noch eine Diazogruppe, Nitrogruppe oder Nitrosogruppe aufweist;
• **n** liegt im Bereich von 0 bis einschließlich 4, mit der Maßgabe, dass die Gruppen R₁ gleich oder verschieden sein können, wenn n 2 bedeutet oder darüber liegt;
• **R₂** bedeutet
- ein Wasserstoffatom;
- eine Alkylgruppe, die ungesättigt, unsubstituiert oder mit einer oder mehreren Gruppen Carboxy, Alkylcarbonyl, Alkoxycarbonyl, Carbamoyl, Mono- oder Dialkylcarbamoyl oder einem oder mehreren stickstoffhaltigen, sauerstoffhaltigen und/oder schwefelhaltigen, gesättigten und/oder ungesättigten Heterocyclen mit 4, 5, 6 oder 7 Atomen substituiert sein kann;
- eine Alkylgruppe, die ungesättigt und in 2-Stellung oder einer Stellung größer 2 mit einer oder mehreren Gruppen Hydroxy, Alkoxy, Amino, Mono- oder Dialkylamino, Thio oder Halogen substituiert sein kann;
- eine Alkylcarbonylgruppe;
- eine Alkoxycarbonylgruppe;
- eine Mono- oder Dialkylcarbamoylgruppe;
- eine Carbamoylgruppe;
- eine Gruppe R₆R₇N-C=NR₅-, worin R₅, R₆ und R₇ ein Wasserstoffatom, eine C₁₋₄-Alkylgruppe oder eine Hydroxyalkylgruppe bedeuten, wobei R₅ vorzugsweise Wasserstoff bedeutet und R₆ und R₇ vorzugsweise unter Wasserstoff oder Methyl ausgewählt sind;
• **R₃** bedeutet:
- ein Wasserstoffatom;
- eine Alkylgruppe, die ungesättigt sein kann;
- eine Hydroxygruppe;
- eine Hydroxyalkylgruppe;
- eine Alkoxygruppe;
- eine Alkoxyalkylgruppe;
- eine Alkylcarbonylgruppe;
- eine Hydroxyalkoxyalkylgruppe;
- eine Aminogruppe;
- eine Monoalkylaminogruppe oder Dialkylaminogruppe;
- eine Aminoalkylgruppe, wobei die Aminogruppe mit Alkyl, Acetyl oder Hydroxyalkyl mono- oder disubstituiert sein kann;
- eine Hydroxy- und Aminoalkylgruppe;
- eine Carboxygruppe;
- eine Carboxyalkylgruppe;
- eine Carbamoylgruppe;
- eine Carbamoylalkylgruppe;
- eine Alkoxycarbonylgruppe;
- eine Mono- oder Dialkylaminocarbonylgruppe;
• **R₄** bedeutet
- eine Alkylgruppe, die ungesättigt sein kann;
- eine Hydroxyalkylgruppe;
- eine Alkoxyalkylgruppe;
- eine Alkylcarbonylgruppe;
- eine Hydroxyalkoxyalkylgruppe;
- eine Aminoalkylgruppe, wobei die Aminogruppe mit Alkyl, Acetyl, Hydroxyalkyl mono- oder disubstituiert sein kann;
- eine Hydroxy- und Aminoalkylgruppe;
- eine Carboxygruppe;
- eine Carboxyalkylgruppe;
- eine Carbamoylgruppe;
- eine Carbamoylalkylgruppe;
- eine Alkoxycarbonylgruppe;
- eine Mono- oder Dialkylaminocarbonylgruppe;
• m liegt im Bereich von 0 bis einschließlich 4, mit der Maßgabe, dass die Gruppen R₄ gleich oder verschieden sein können, wenn m 2 bedeutet oder darüber liegt.

2. Zusammensetzung nach Anspruch 1, wobei R₁ eine Alkylgruppe, Hydroxyalkylgruppe oder Aminoalkylgruppe, Alkoxygruppe oder Hydroxyalkoxygruppe ist.

3. Zusammensetzung nach Anspruch 1 oder 2, wobei R₁ unter Methyl, Hydroxymethyl, 2-Hydroxyethyl, 1,2-Dihydroxyethyl, Methoxy, 2-Hydroxyethoxy ausgewählt ist.

4. Zusammensetzung nach einem der vorhergehenden Ansprüche, wobei n 0 oder 1 bedeutet.

5. Zusammensetzung nach einem der vorhergehenden Ansprüche, wobei R₂ unter Wasserstoff, einer Alkylgruppe, einer Alkylgruppe, die mit einem stickstoffhaltigen und/oder sauerstoffhaltigen, gesättigten oder ungesättigten Heterocyclus mit 4, 5 oder 6 Atomen substituiert ist, einer Alkoxycarbonylgruppe, einer Alkylgruppe, die in 2-Stellung oder einer Stellung größer 2 mit einer oder mehreren Hydroxygruppen substituiert ist.

6. Zusammensetzung nach einem der vorhergehenden Ansprüche, wobei R₂ 2-Hydroxyethyl, 3-(1-Pyrrolidinyl)propyl, Methyl, Acetyl oder Wasserstoff bedeutet.

7. Zusammensetzung nach einem der vorhergehenden Ansprüche, wobei R₃ unter Wasserstoff, Alkyl, einer Alkylgruppe, die mit einer oder mehreren Hydroxygruppen substituiert ist, einer Alkylgruppe, die mit einer oder mehreren Aminogruppen substituiert ist, Carboxy ausgewählt ist.

8. Zusammensetzung nach einem der vorhergehenden Ansprüche, wobei R₃ unter Wasserstoff, Hydroxy, Carboxy, Amino, Hydroxymethyl, Aminomethyl ausgewählt ist.

9. Zusammensetzung nach einem der vorhergehenden Ansprüche, wobei R₄ unter Wasserstoff, Alkyl, einer Alkylgruppe, die mit einer oder mehreren Hydroxygruppen substituiert ist, einer Alkylgruppe, die mit einer oder mehreren Aminogruppen substituiert ist, Carboxy ausgewählt ist.

10. Zusammensetzung nach einem der vorhergehenden Ansprüche, wobei R₄ unter Wasserstoff ausgewählt ist.

11. Zusammensetzung nach einem der vorhergehenden Ansprüche, wobei die Oxidationsbase der Formel (I) ausgewählt ist unter:
4-(4-Methyl-[1,4]diazepan-1-yl)-phenylamin
4-[1,4]Diazepan-1-yl-phenylamin
4-[4-(3-Pyrrolidin-1-yl-propyl)-[1,4]diazepan-1-yl]-phenylamin
2-[4-(4-Aminophenyl)-[1,4]diazepan-1-yl]-ethanol
1-[4-(4-Aminophenyl-[1,4]diazepan-1-yl]-ethanon
4-(4-Aminophenyl)-[1,4]diazepan-1-carboxamidin
4-(4-Aminophenyl)-N,N-dimethyl-[1,4]diazepan-1-carboxamidin
1-(4-Aminophenyl)-4-methyl-[1,4]diazepan-6-ol
1-(4-Aminophenyl)-4-methyl-[1,4]-diazepan-6-ylamin
1-(4-Aminophenyl)-4-(3-pyrrolidin-1-yl-propyl)-[1,4]diazepan-6-ol
1-(4-Aminophenyl)-4-(3-pyrrolidin-1-yl-propyl)-[1,4]diazepan-6-ylamin
1-(4-Aminophenyl)-4-(2-hydroxyethyl)-[1,4]diazepan-6-ol
2-[6-Amino-4-(4-aminophenyl)-[1,4]diazepan-1-yl]-ethanol
2-[4-(4-Aminophenyl)-6-hydroxymethyl-[1,4]diazepan-1-yl]-ethanol
2-Methyl-4-(4-methyl-[1,4]diazepan-1-yl)-phenylamin
4-[1,4]Diazepan-1-yl-2-methylphenylamin
2-Methyl-4-[4-(3-pyrrolidin-1-yl-propyl)-[1,4]diazepan-1-yl]-phenylamin
2-[4-(4-Amino-3-methylphenyl)-[1,4]diazepan-1-yl]-ethanol
1-[4-(4-Amino-3-methylphenyl)-[1,4]diazepan-1-yl]-ethanon
4-(4-Amino-3-methylphenyl)-[1,4]diazepan-1-carboxamidin
4-(4-Amino-3-methylphenyl)-N,N-dimethyl-[1,4]diazepan-1-carboxamidin
1-(4-Amino-3-methylphenyl)-4-methyl-[1,4]diazepan-6-ol
1-(4-Amino-3-methylphenyl)-4-methyl-[1,4]diazepan-6-ylamin
1-(4-Amino-3-methylphenyl)-4-(3-pyrrolidin-1-yl-propyl)-[1,4] diazepan-6-ol
1-(4-Amino-3-methylphenyl)-4-(3-pyrrolidin-1-yl-propyl)-[1,4]diazepan-6-ylamin
1-(4-Amino-3-methylphenyl)-4-(2-hydroxyethyl)-[1,4]diazepan-6-ol
2-[6-Amino-4-(4-amino-3-methylphenyl)-[1,4]diazepan-1-yl]-ethanol
2-[4-(4-Amino-3-methylphenyl)-6-hydroxymethyl-[1,4]diazepan-1-yl]-ethanol.

12. Zusammensetzung nach einem der vorhergehenden Ansprüche, wobei die Oxidationsbase(n) der Formel (I) in einer Menge von 0,001 bis 10 % und vorzugsweise 0,005 bis 6 % enthalten sind.

13. Zusammensetzung nach einem der vorhergehenden Ansprüche, die einen oder mehrere Kuppler enthält, die unter den *m*-Phenylendiaminen, *m*-Aminophenolen, *m*-Dihydroxybenzolen, Naphthalinkupplern und heterocyclischen Kupplern und deren Additionssalzen ausgewählt sind.

14. Zusammensetzung nach einem der vorhergehenden Ansprüche, die eine zusätzliche Oxidationsbase enthält, die unter den *p*-Phenylendiaminen, Bisphenylalkylendiaminen, p-Aminophenolen, o-Aminophenolen, heterocyclischen Basen und deren Additionssalzen ausgewählt ist.

15. Zusammensetzung nach einem der vorhergehenden Ansprüche, die ferner einen Direktfarbstoff enthält.

16. Verfahren zum oxidativen Färben von Keratinfasern, **dadurch gekennzeichnet, dass** auf die Fasern mindestens eine Zusammensetzung nach einem der Ansprüche 1 bis 15 aufgetragen und die Farbe mit Hilfe eines Oxidationsmittels gebildet wird.

17. Verfahren nach Anspruch 16, wobei das Oxidationsmittel unter Wasserstoffperoxid, Harnstoffperoxid, Alkalimetallbromaten, Persalzen, Persäuren und Oxidasen ausgewählt ist.

18. Verfahren nach einem der Ansprüche 16 oder 17, wobei das Oxidationsmittel bei der Anwendung mit der Zusammensetzung nach einem der Ansprüche 1 bis 15 vermischt wird.

19. Verfahren nach einem der Ansprüche 16 oder 17, wobei das Oxidationsmittel in Form einer oxidierenden Zusammensetzung gleichzeitig mit der Zusammensetzung nach einem der Ansprüche 1 bis 15 oder getrennt davon anschließend auf die Fasern aufgebracht wird.

20. Vorrichtung mit mehreren Abteilungen oder "Kit" zum Färben mit mehreren Abteilungen, wobei eine erste Abteilung eine Zusammensetzung nach einem der Ansprüche 1 bis 15 und eine zweite Abteilung eine oxidierende Zusammensetzung enthält.

21. Farbiges Produkt, das durch oxidative Kondensation einer Zusammensetzung nach einem der Ansprüche 1 bis 15 erhältlich ist.

22. *p*-Phenylendiaminderivate der Formel (I) nach einem der Ansprüche 1 bis 11, wobei 4-(4-Methyl-[1,4]diazepan-1-yl)-phenylamin, 3-Cyano-4-(4-*t*-butoxycarbonyl-1,4-cyclodiazacycloheptan)-anilin, 4-(4-*t*-Butoxycarbonyl-1,4-diazacycloheptan)-anilin und 3-Fluor-4-(N-*t*-butoxycarbonylhomopiperazinyl)-anilin ausgenommen sind.
